# FASCICULE DE BREVET EUROPEEN

(11) **EP 0 525 098 B1**
(45) Date de publication et mention de la délivrance du brevet: **21.09.1994**
(21) Numéro de dépôt: 91908771.8
(22) Date de dépôt: 11.04.1991
(51) Int. Cl.: A61F 9/00

(54) **KERATOTOME DESTINE A LA REALISATION D'INCISIONS ARCIFORMES**
KERATOTOM ZUM REALISIEREN VON PREISBOGENFÖRMIGEN EINSCHNITTEN
KERATOME FOR MAKING ARC-SHAPED INCISIONS

(30) Priorité: 12.04.1990 FR 9004722
(43) Date de publication de la demande: 03.02.1993
(73) Titulaire: Hanna, Khalil, F-75007 Paris (FR)
(72) Inventeur: Hanna, Khalil, F-75007 Paris (FR)
(74) Mandataire: Robert, Jean-Pierre
(86) Numéro de dépôt international: FR9100300
(87) Numéro de publication internationale: WO9116023

(56) Documents cités:
- EP-A- 0 047 190
- US-A- 4 423 728
- US-A- 4 608 977
- US-A- 4 648 400
- ARCH. OPHTALM. Vol. 106, August 1988, US pages 1130-1135; DUFFEY ET AL.: "Paired arcuate keratotomy" see page 1134, column 2, line 46 - column 3, line 2

## Description

La présente invention concerne un kératotome destiné à la réalisation d'incisions arciformes dans une cornée, en vue de la correction de l'astigmatisme.

On sait que l'astigmatisme d'un oeil résulte de ce que deux méridiens mutuellement perpendiculaires de la face antérieure de la cornée n'ont pas la même courbure et une méthode connue de correction de l'astigmatisme consiste à pratiquer dans la cornée, pependiculairement au méridien le plus courbe, c'est-à-dire qui a le rayon de courbure le plus petit, deux incisions disposées respectivement de part et d'autre de la zone optique.

Merlin ("Curved keratotomy procedure for congenital astigmatism". Journal of Refractive Surgery 1987 ; 3:92-97) a pratiqué des incisions arciformes en vue de la correction de l'astigmatisme, en utilisant un couteau micrométrique tenu manuellement, et en se guidant visuellement sur une marque réalisée au prélable au moyen d'un marqueur circulaire.

Il est toutefois difficile de réaliser des incisions de profondeur uniforme au moyen d'un couteau simplement tenu à la main, et ce d'autant plus que le diamètre où ces incisions sont situées est petit.

De ce fait, de telles incisions arciformes sont peu utilisées.

Le but de la présente invention est de permettre la réalisation d'incisions arciformes suffisamment précises pour aboutir à des résultats, indépendamment de l'habileté du chirurgien.

La présente invention vise à résoudre le but fixé par des moyens mécaniques et, à cet effet, elle propose un kératotome destiné à la réalisation d'une incision dans une cornée et comportant à cet effet, comme il est connu dans le domaine des trépans de kératoplastie, notamment par le document US 4 423 728:
- un support extérieur tubulaire formant enveloppe, présentant un axe déterminé et comportant, pour son application une cornée, une partie de pied annulaire de révolution autour de l'axe du support et délimitant une surface géométrique de référence définie comme la forme que présente la cornée lorsque le support est appliqué sur celle-ci par sa partie de pied,
- un corps intérieur tubulaire disposé coaxialement à l'intérieur du support, en retrait vers l'intérieur du support par rapport à la surface de référence,
- des moyens de guidage du corps en rotation autour de l'axe du support par rapport au support,
- des moyens d'entraînement du corps en rotation autour de l'axe du support par rapport au support,
- un couteau disposé à l'intérieur du corps, suivant une direction propre, et présentant dans un sens déterminé de sa direction propre une pointe coupante,
- des moyens de liaison entre le couteau et le corps, comportant eux-mêmes des moyens pour déplacer de façon réglée le couteau en translation suivant sa direction propre, par rapport au corps, entre une position de repos, dans laquelle il est placé en retrait vers l'intérieur du support par rapport à la surface de référence et dans laquelle la pointe est tournée vers ladite surface de référence, et une position de coupe, dans laquelle la pointe forme une saillie à l'extérieur du support par rapport à la surface de référence, de façon décalée par rapport à l'axe du support,
et, conformément à l'invention, en vue de la réalisation d'incisions arciformes de position angulaire déterminée et de longueur angulaire déterminée
- des moyens de détermination de la position angulaire relative du support et du corps comprenant des butées définissant deux positions angulaires relatives limites du support et du corps et portées respectivement par le support et par le corps.

On conçoit aisément qu'un tel kératotome autorise la réalisation d'incisions en forme d'un arc de cercle rigoureux, concernant de ce fait une zone dans laquelle la cornée présente une épaisseur sensiblement constante, et permette de maîtriser sans difficulté deux paramètres importants, à savoir la profondeur d'incision, réglée par réglage de la saillie que la pointe du couteau forme à l'extérieur du corps en position de coupe, et la longueur angulaire de l'incision.

D'autres caractéristiques et avantages d'un kératotome selon l'invention ressortiront de la description ci-dessous, relative à un exemple non limitatif de réalisation, ainsi que des dessins annexés qui font partie intégrante de cette description.
- la figure 1 montre un kératotome selon l'invention, pour moitié en élévation latérale et pour moitié en coupe par un plan repéré en I-I à la figure et incluant l'axe de rotation relative du corps intérieur et du support extérieur, lequel axe coïncide avec l'axe visuel lors de l'utilisation du kératotome.
- la figure 2 montre une vue partielle du kératotome, en plan, c'est-à-dire tel qu'il apparaît à l'opposé de sa partie de pied, parallèlement à l'axe de rotation relative du corps intérieur et du support extérieur, dans un sens repéré par une flèche II à la figure 1 ; pour des raisons de clarté, on a omis sur cette figure les couteaux, les porte-couteaux ainsi que le détail des moyens d'entraînement en rotation du corps par rapport au support.
- la figure 3 montre une vue, en coupe par deux demi-plans incluant l'axe de rotation relative du corps intérieur et du corps extérieur et repérés en III-III à la figure 2, la bague portée par le support, de façon réglable en position angulaire autour de l'axe de rotation relative du corps et du support, et portant elle-même les butées prévues sur le support pour limiter la rotation du corps.
- la figure 4 montre, vu en coupe par un plan incluant l'axe de rotation du corps par rapport au support, tel que le plan I-I de la figure 2, un premier mode de réalisation des moyens de repérage du positionnement du kératotome par rapport à la cornée.
- la figure 5 montre une vue de ces moyens en plan, par exemple dans le sens repéré par la flèche II à la figure 1.
- les figures 6 et 7 montrent, respectivement, en une vue en plan dans le sens repéré par la flèche II à la figure 1 et en coupe par un plan repéré en IX-IX à la figure 2 et incluant l'axe de rotation du corps, des moyens de repérage d'un axe d'astigmatisme.
- la figure 8 montre, en une vue dans un sens repéré par une flèche X à la figure 1, un détail des moyens micrométriques d'écartement entre des zones respectives du porte-couteau et du corps, pour permettre le réglage de la valeur du décalage de la pointe d'un couteau par rapport à l'axe de rotation relative du corps et du support.
- la figure 9 montre une vue de détail d'un couteau, dans un sens repéré par une flèche XI à la figure 1.
- la figure 10 montre, en une vue dans un sens repéré par une flèche XII à la figure 1, un détail des moyens pour déplacer de façon réglée un couteau en translation suivant sa direction propre, c'est-à-dire pour régler la valeur de la saillie que la pointe du couteau forme à l'extérieur du support, par rapport à la surface de référence, en position de coupe.
- la figure 11 montre un extrait de la figure 1, à plus grande échelle.

Le kératotome illustré est un type comportant deux couteaux, mais il est bien entendu que l'on ne sortirait pas du cadre de la présente invention en réalisant conformément aux enseignements de celle-ci un kératotome comportant un seul couteau, ou éventuellement plus de deux couteaux.

En vue de sa préhension manuelle par le chirurgien et de son positionnement sur la face antérieure 1 de la cornée 2 à inciser, le kératotome illustré 3 comporte un support extérieur 4 présentant la forme générale d'une paroi tubulaire tronconique de révolution autour d'un axe 5 et enveloppant un corps intérieur ou chariot 6 présentant également la forme d'une paroi tubulaire de révolution autour de l'axe 5, montée tournante dans le support autour de cet axe 5 à l'exclusion de toute autre possibilité de déplacement relatif.

Le support extérieur 4 diverge d'une partie de pied 7 destinée à s'appliquer sur la face antérieure 1 de la cornée 2 à inciser, autour de l'emplacement des incisions à réaliser, jusqu'à une partie de tête 8 destinée à sa préhension par la main non dominante du chirurgien.

La paroi constituant le support extérieur 4 dans sa partie de pied 7 comporte une cavité 9 annulaire, de révolution autour de l'axe 5 et s'ouvrant dans la partie de pied 7 par une fente annulaire 10, délimitée par deux portées 11, 12 annulaires de révolution autour de l'axe 5 et se situant sur une même surface de référence 13. La surface de référence 13 est choisie de façon à correspondre à la majorité des faces antérieures 1 de cornée 2. Un effet d'aspiration vers l'intérieur de la cavité 9, par la fente 10, résulte du raccordement de cette cavité 9 à des moyens 14 permettant d'y établir une dépression, raccordés à la cavité 9 par un embout 15 de raccordement de celle-ci, extérieur au support 4, et par un conduit souple 16.

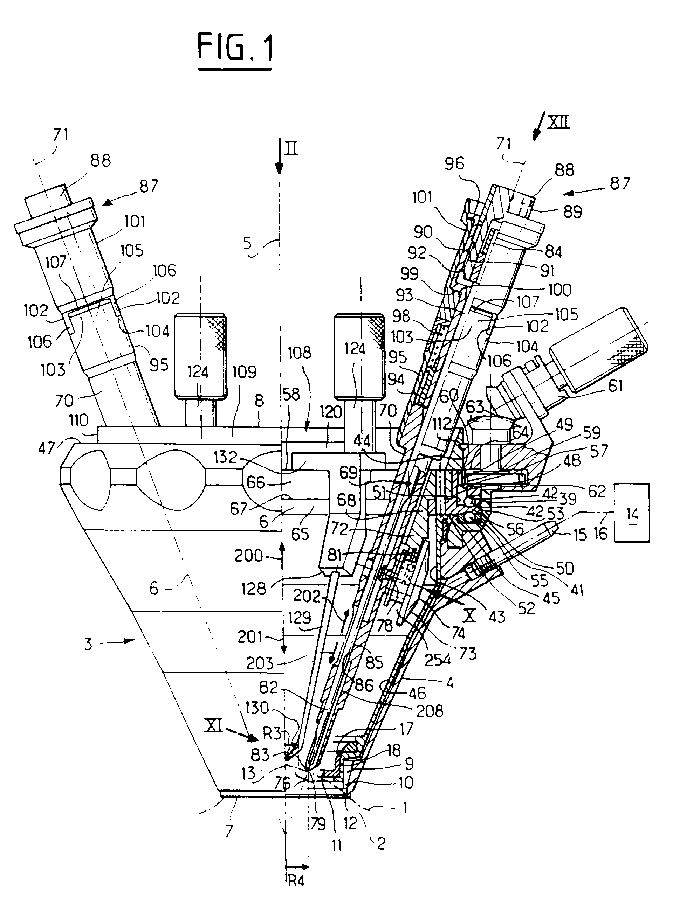

De préférence, la portée 11 la plus proche de l'axe 5 est constituée par une pièce annulaire 17 rapportée à rotation sur le support 4, dans une gorge 18 annulaire de ce dernier, tournée vers l'axe 5. Cette gorge est délimitée par deux pièces démontables afin de permettre de changer la pièce 17 illustrée aux figures 1, 4, 5

La pièce 17 ou toute autre pièce susceptible de leur être substituée, comporte une jupe périphérique 20, 21, annulaire de révolution autour de l'axe 5 avec une forme étroitement complémentaire de celle de la gorge 18. A l'intérieur de la jupe 20 ou 21, la pièce 17 présente une paroi délimitée par une face 22, 23 plane, supérieure, perpendiculaire à l'axe 5 et présentant, sur l'autre face, des formes déterminées affleurant la surface de référence 13.

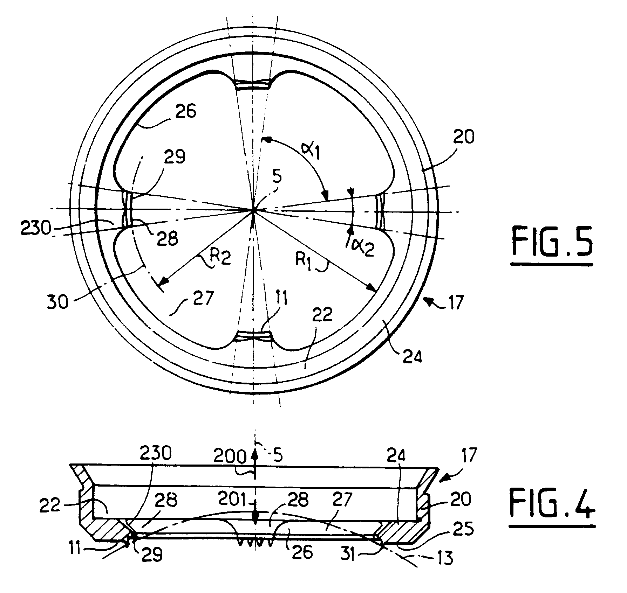

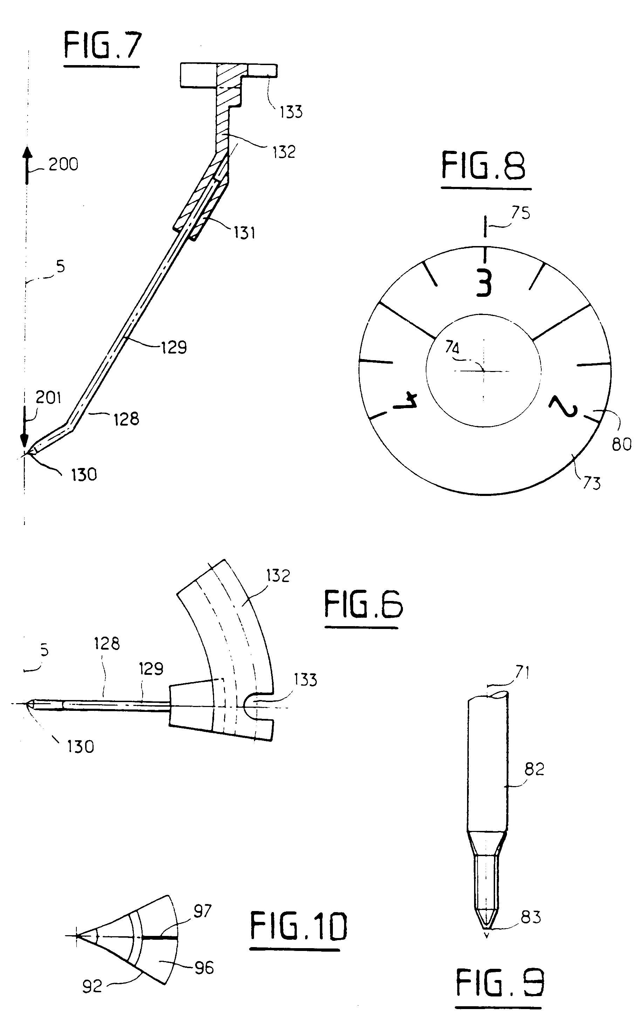

Ainsi, comme le montrent plus particulièrement les figures 4 et 5, la pièce 17 présente une paroi transversale en forme de couronne 24, limitée par deux faces planes 22, 25, et un chant annulaire 26. Quatre zones 27 disposées suivant un même cylindre géométrique non référencé, de rayon R₁, de longueur angulaire α₁ proche de 90°, alternent avec quatre zones 28 de longueur angulaire α₂ de l'ordre de quelques degrés. Chacune de ces zones 28 porte sur sa face 25 une arête 29 en arc de cercle 30 de rayon R₂ inférieur à R₁ et en pratique supérieur à celui de la zone claire de la cornée.

Les quatre arêtes 29 permettent un positionnement précis du kératome 3 sur la cornée 2, préalablement pourvue d'un marquage circulaire centré sur l'axe visuel. Les quatre arêtes 29 affleurent la surface de référence 13 pour constituer ensemble la portée 11 de contact avec la face antérieure 1 de la cornée 2, sur le marquage circulaire de même rayon que le cercle 30.

La pièce 17 peut comporter en outre, en saillie sur sa face 25 à proximité immédiate de chaque arête 29, un groupe de dents 31 empêchant un glissement du kératome sur la face antérieure 1 de la cornée ; la pièce 17 est par ailleurs entièrement située en retrait par rapport à la surface de référence 13.

Le support extérieur 4 comporte une autre gorge 39 annulaire intérieure dans sa zone de tête 8 avec un fond 40 de diamètre aussi grand que possible compte-tenu des dimensions de la partie de tête 8. La gorge 39 comporte deux flancs plans 41, 42, perpendiculaires à l'axe 5. Les flancs 41 et 42 débouchent sur des faces respectives 43, 44 du support extérieur 4, lesquelles sont cylindriques de révolution autour de l'axe 5 vers lequel elles sont tournées, par l'intermédiaire d'un rebord 45, pour le flanc 41 et directement pour le flanc 42. Sous la face 43, le support 4 comporte une face 46 tronconique convergeant vers sa partie de pied 7. Le support extérieur 4 est limité en partie supérieure par une face 47 plane.

Le support 4 qui vient d'être décrit est avantageusement constitué de plusieurs pièces assemblées mutuellement par des moyens permettant de les dissocier à volonté, de telle sorte qu'on puisse le nettoyer aisément. En particulier, les parties de tête 8 et de pied 7, bien que mutuellement solidaires, sont dissociables à volonté au niveau de l'épaulement 45 de façon à permettre l'accès à des moyens de réglage qui seront décrits plus loin.

Le support extérieur 4 coopère par la gorge 39 et la face 44 avec le corps intérieur 6 pour assurer le guidage de ce dernier en rotation autour de l'axe 5 sans aucune possiblité de mouvement relatif.

A cet effet, le corps 6 porte une face 48 cylindrique de diamètre légèrement inférieur à celui de la face 44, en regard de laquelle elle est placée sans contact mutuel. Cette face 48 porte deux épaulements 49 et 50 qui forment une saillie radiale et qui sont placés respectivement en regard de la face 44 et en regard de la gorge 39, dans laquelle s'engage l'épaulement 50. L'épaulement 50 est délimité par des faces annulaires planes 51, 52 et par une face périphérique extérieure 53 cylindrique glissant sur le fond 40 de la gorge 39.

Les faces 52 et 51 de l'épaulement 50 sont en appui respectivement contre les flancs 41 et 42 par l'intermédiaire d'un roulement à billes 55, 56. L'épaulement 49 est délimité par des faces annulaires planes 57, 58 et une face 59 de forme générale cylindrique de révolution autour de l'axe 5, avec un diamètre sensiblement identique à celui de la face 44 de façon à établir par contact glissant un autre guidage du corps 6 et du support 4 en rotation autour de l'axe 5. La face 59 est creusée de dents d'engrenage 60 régulièrement réparties angulairement autour de l'axe 5 de façon à coopérer avec des moyens manuels d'entraînement 61 de conception connue, par exemple décrite dans le document EP 0 047 190, qui coopèrent avec les dents d'engrenage 60 du corps 6 par l'intermédiaire d'un pignon 62 monté à rotation sur le support 4 autour d'un axe 63 parallèle à l'axe 5, lequel pignon 62 traverse localement la face 44 du support 4, vers l'axe 5, par une lumière 64 de cette face 44.

On remarquera que du fait des dispositions qui viennent d'être décrites et lorsque l'on sépare la partie de pied 7 de la partie de tête 8 au niveau de l'épaulement 45, le corps 6 du kératotome 3 reste assemblé à la partie de tête 8, qui porte également les moyens d'entraînement manuel 61.

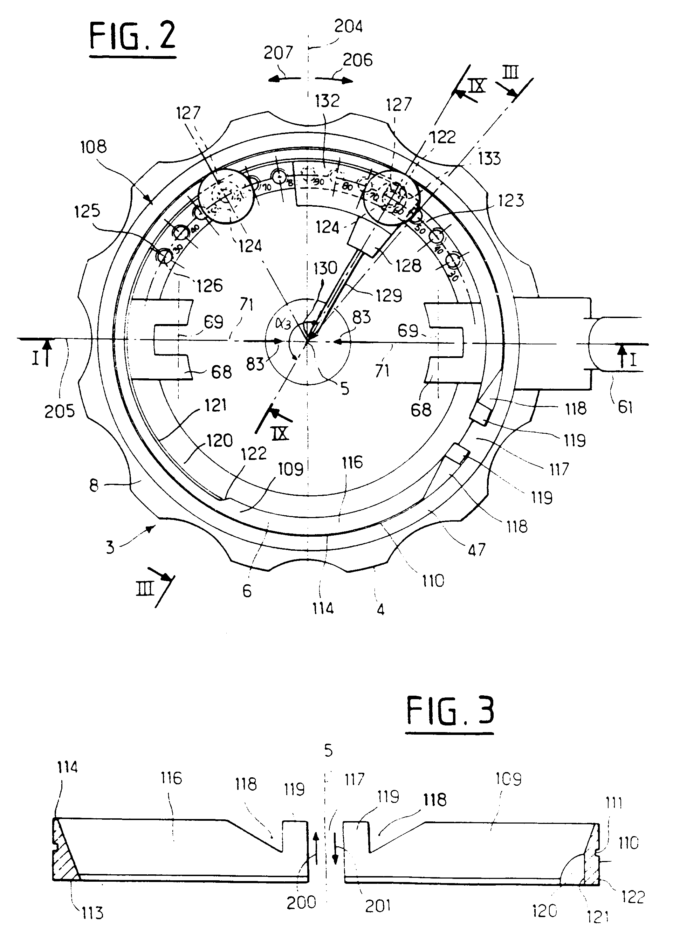

Par des faces 65, 66, 67 délimitant un épaulement intérieur, le corps 6 porte de façon solidaire mais de préférence amovible en vue des opérations de maintenance, deux chapes identiques 68, diamétralement opposées. Chacune de ces chapes 68 assure le montage tourillonnant d'un porte-couteau respectif 70 autour d'un axe d'articulation 69 par rapport au corps 6. Les deux axes 69 sont situés perpendiculairement à un même plan 205 incluant l'axe 5 et se confondant avec le plan repéré en I-I à la figure 2 et symétriquement l'un de l'autre par rapport à un plan 204, incluant également l'axe 5 mais perpendiculaire au plan 205, et sont situés à la fois aussi loin que possible de l'axe 5 et aussi près que possible de la face 58 de l'épaulement 49 du corps 6, c'est-à-dire de la face 47 de la partie de tête 48 du support 4 afin d'être aussi éloignés que possible de la partie de pied 7 de celui-ci.

Dans le plan 205 incluant l'axe 5 et perpendiculaire aux axes 69, le porte-couteau 70 présente une direction moyenne propre 71 perpendiculaire à l'axe 69. On peut ainsi l'orienter par rapport à l'axe 5 en faisant pivoter le porte-couteau 70 autour de l'axe 69, par rapport à la chape 68 et au corps 6. En pratique, la direction moyenne propre 71 de chaque porte-couteau 70 coupe la surface de référence 13 à l'intérieur d'une zone annulaire 76 de celle-ci, de révolution autour de l'axe 5 avec un rayon minimal R₃ inférieur au rayon R₂ précité mais néanmoins supérieur à celui de la zone claire d'une cornée 2.

Des moyens sont prévus pour immobiliser de façon réglée chaque porte-couteau 70 en rotation autour de l'axe 69.

A cet effet, chaque chape 68 présente une zone 72 formant une saillie approximativement parallèle à l'axe 5, par rapport à la face 52 de l'épaulement 50 vers la partie de pied 7 du support 4, cette zone 72 étant en retrait vers l'intérieur du support 4 par rapport à la surface de référence 13 et n'entrant en contact avec aucune partie du support 4 bien que cette zone 72 soit située plus loin de l'axe 5 que le porte-couteau 70.

Par cette zone 72, la chape 68 porte une couronne 73, tournante autour d'un axe 74 mais sans possibilité de translation suivant cet axe 74 ; l'axe 74 est situé dans le plan 205, décalé par rapport à l'axe 69, vers la partie de pied 7 du support 4, et perpendiculaire à la direction propre 71 du porte-couteau 70, par rapport auquel cet axe 74 est fixe.

La couronne 73 est ainsi disposée du côté de la zone 72 opposé au côté de celle-ci tournée vers l'axe 5, dans une position dégagée par la partie de pied 7 du support 4 lorsque celle-ci est séparée de la partie de tête 8 de façon à permettre alors sa rotation manuelle. Comme le montre la figure 8, cette couronne 73 porte des graduations 80 visibles lorsque la partie de pied 7 est ainsi séparée de la partie de tête 8, ces graduations étant exprimées par exemple en mm, et correspondant à des diamètres d'incision. De façon complémentaire, la zone 72 de la chape 68 ou le porte-couteau 70 lui-même porte un repère 75, par exemple sous la forme d'un simple trait.

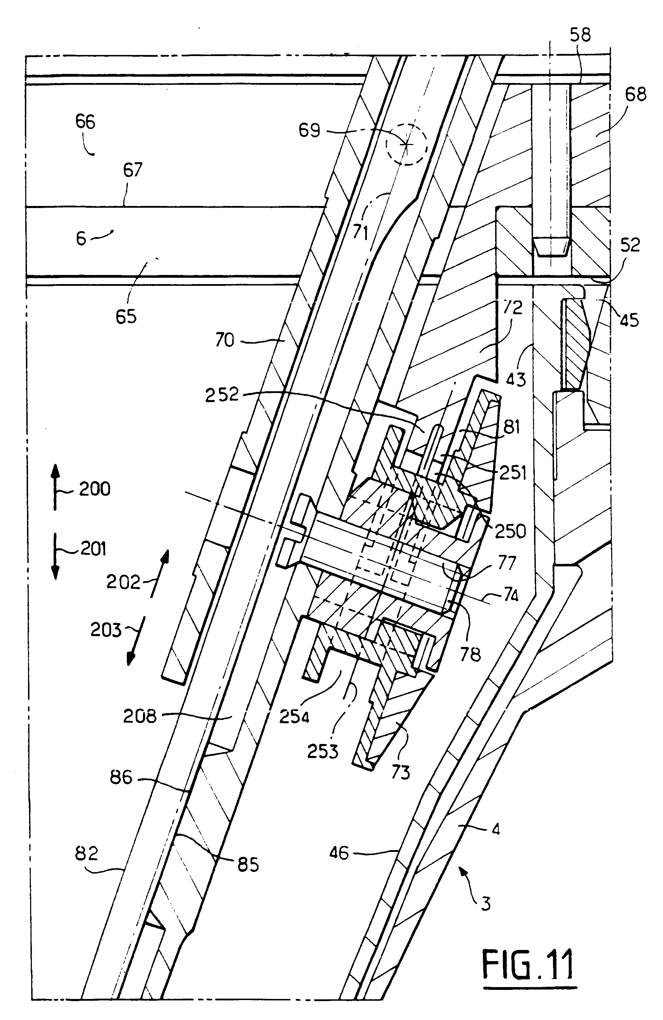

Vers l'axe 74, comme le montre la figure 11, la couronne 73 présente un taraudage micrométrique 77, en prise avec une vis coaxiale 78 montée sur le porte-couteau 70 de façon solidaire. En tournant la couronne 73, on peut régler l'écartement entre le porte-couteau 70 et la zone 72 de la chape 68 au niveau de l'axe 74, c'est-à-dire régler l'orientation de la direction propre 71 par rapport à l'axe 5 et, en particulier, l'écartement ou décalage R₄, intermédiaire entre R₂ et R₃, séparant de l'axe 5 le point 79 d'intersection de cette direction propre 71 avec la surface de référence 13, dans les limites de la zone 76 de la surface de référence 13. Cet écartement ou décalage R₄ détermine un rayon d'incision.

Pour autoriser un tel mouvement, le montage de la couronne 73 sur la zone 72 de la chape 68 s'effectue, comme le montre la figure 11, par engagement d'une gorge 254, annulaire de révolution autour de l'axe 74, que présente la couronne 73, dans une glissière 81 de la zone 72 de la chape 68. Cette glissière 81 présente la forme d'une fourche s'ouvrant vers la partie de pied 7 et comportant deux bras 250 symétriques par rapport au plan 205 et s'engageant dans la gorge 254 de part et d'autre de l'axe 74. Chacun des bras 250 se dédouble lui-même, vers la partie de pied 7, en deux branches 251, 252 qui sont situées de part et d'autre d'un plan 253 approximativement perpendiculaire à l'axe 74 et prennent appui de façon antagoniste, parallèlement à cet axe 74, dans la gorge 254. Les branches 252 les plus proches de l'axe 5 sont rigides mais les branches 251 les plus éloignées de l'axe 5 sont dimensionnées, de façon aisément déterminable par un Homme du métier et en fonction du matériau constitutif de la chape 68, convenablement choisi à cet effet par cet Homme du métier, de façon à présenter une élasticité telle que les deux branches 251 et 252 soient en permanence retenues en précontrainte, dans le sens d'un rapprochement mutuel dans la gorge 254 ; un tel montage, tout en empêchant tout déplacement de la couronne 73 parallèlement à l'axe 74 par rapport à la zone 72 de la chape 68, et plus précisément par rapport aux branches 252 les plus rigides des bras 250, autorise un débattement de la couronne 73 par rapport à l'axe 69 ainsi qu'un basculement de la couronne 73 par rapport à la zone 72 de la chape 68.

Chaque porte-couteau 70 présente une forme générale tubulaire, de révolution autour de sa direction propre 71, et délimite intérieurement un canal 208 recevant, en coulissement suivant cette direction propre 71, un couteau 82 s'étendant de façon rectiligne d'une pointe extrême 83 en forme de lancette, comme le montre la figure 9, tournée vers la partie de pied 7, à une extrémité de manoeuvre 84 placée en saillie hors du corps 6 et du support 4 au niveau de la partie de tête 8 de ce dernier. Des méplats respectifs 85, 86 du couteau 82 et du porte-couteau 70 empêchent une rotation autour de la direction propre 71, qui constitue également pour le couteau 82 une direction moyenne propre passant par sa pointe extrême 83.

Au niveau de son extrémité 84, chaque couteau 82 coopère avec le porte-couteau 70 correspondant par l'intermédiaire de moyens 87 permettant de déplacer de façon réglée le couteau 82, en translation suivant la direction propre 71, par rapport au porte-couteau 70, entre deux positions limites :
- une position de repos dans laquelle le couteau est illustré à la figure 1 et dans laquelle il est placé en retrait vers l'intérieur du corps 6 et du support 4, dans un sens 202 de la direction propre 71, par rapport à la surface de référence 13 que la pointe 83 affleure toutefois,
- une position de coupe que l'on a schématisée en trait mixte à la figure 1 et dans laquelle la pointe 83 forme une saillie à l'extérieur du corps 6 et du support 4, dans un sens 203 opposé au sens 202, par rapport à la surface de référence 13 que le couteau 82 coupe alors en respectant le décalage réglé R₄ par rapport à l'axe 5, l'amplitude de cette saillie pouvant être réglée par action sur les moyens 87, indépendamment pour l'un et l'autre des couteaux 82.

A cet effet, par son extrémité 84, chaque couteau 82 porte un bouton de réglage 88, placé en saillie hors du porte-couteau 70 à l'opposé de la pointe 83. Ce bouton 88 est monté tournant autour de la direction propre 71 par rapport au couteau 82 et solidaire en translation par rapport au porte-couteau 70 suivant la direction propre 71. Le bouton 88 porte des graduations 89 exprimées par exemple en dixièmes de millimètres, ces graduations correspondant à la saillie que la pointe 83 du couteau 82 forme par rapport à la surface de référence 13 en position de coupe.

Le bouton 88 porte un filetage 90 par lequel il est en prise avec un taraudage 91 d'un fourreau 92 monté en translation suivant la direction propre 71 aussi bien par rapport au bouton de réglage 88 que par rapport au porte-couteau 70. Le fourreau 92 enveloppe ainsi localement le bouton 88, au niveau de son filetage 90 ainsi que, localement, un tronçon 93 du couteau 82 situé entre la partie de tête 8 du support 4 et l'extrémité 84.

Le fourreau 92 peut se déplacer en translation suivant la direction propre 71 par rapport au porte-couteau 70, de même que par rapport au bouton 88 et au couteau 92, mais il est calé en rotation sur le porte-couteau 70, par un clavetage 94, dans une zone extrême 95 du porte-couteau 70 enveloppant le fourreau 92, en saillie hors du support 4 par la partie de tête 8 de celui-ci, dans le sens 202, entre l'extrémité 84 du couteau 82 et l'axe 69.

Pour coopérer avec les graduations 89 du bouton de réglage 88, le fourreau 92 porte, sur une couronne 96 entourant le bouton de manoeuvre 88, un repère 97 par exemple sous la forme d'un trait radial en référence à la direction propre 71, comme le montre la figure 10, ce qui permet d'assurer un repérage de la position angulaire relative du bouton de réglage 88 et du fourreau 92, c'est-à-dire également du porte-couteau 70, autour de la direction propre 71, c'est-à-dire de permettre d'afficher une valeur réglée de la saillie que la pointe 83 du couteau 82 forme par rapport à la surface de référence 13 lorsque le couteau 82 est en position de coupe.

Par sa zone extrême 95, le porte-couteau 70 enveloppe extérieurement le fourreau 92 et un ressort de compression 98 interposé entre-eux à ce niveau, précontraint suivant la direction propre 71, tend à déplacer élastiquement le fourreau 92 à la translation suivant la direction propre 71, par rapport au porte-couteau 70, vers l'axe 69 et vers l'intérieur du support 4, c'est-à-dire dans le sens 203.

Le fourreau 92 présente extérieurement, entre la zone extrême 95 du porte-couteau 70 et le bouton de réglage 88, un épaulement annulaire 99. Cet épaulement 99 est tourné dans le sens 203 vers l'axe 69 et s'appuie vers ce dernier sur un épaulement opposé 100 intérieur d'un bouton de déclenchement 101 enveloppant le fourreau 92 en dehors de la zone extrême 95 du porte-couteau 70, avec possibilité de rotation du bouton de déclenchement 101 autour de la direction propre 71 par rapport au fourreau 92 ainsi que de translation relative suivant cette direction propre 71, toutefois dans les limites autorisées par le contact mutuel des épaulements 99 et 100, d'une part, et la compression maximale du ressort 98, d'autre part.

L'un vers l'autre, le bouton de déclenchement 101 et le porte-couteau 70, dans sa zone 95, présentent eux-mêmes des moyens de butée mutuelle suivant la direction propre 71, autorisant deux positions relatives stables, suivnt cette direction propre 71, selon la position angulaire relative autour de cette direction propre.

Plus précisément, vers la zone extrême 95 du porte-couteau 70, dans le sens 203, le bouton de déclenchement 101 présente une alternance de nervures 102 et de rainures 103 radiales, les nervures 102 présentant dans le ses 203 des méplats extrêmes 104 perpendiculaires à la direction propre 71. La zone extrême 95 du porte-couteau 70 présente quant à elle vers le bouton de déclenchement 101, dans le sens 202, une alternance de nervures 105 et de rainures 106 radiales complémentaires des rainures 103 et des nervures 102, les nervures 105 présentant, dans le sens 202, des méplats extrêmes 107 plans, perpendiculaires à la direction propre 71.

Le couteau 82, le porte-couteau 70, le bouton de réglage 88, le fourreau 92, le bouton de déclenchement 101 sont dimensionnés, d'une façon aisément déterminable par un Homme du métier, de telle sorte que :
- en plaçant les méplats respectifs 104 et 107 des nervures 102 et 105 en appui mutuel suivant la direction propre 71 et en orientant le bouton de réglage 88 de telle sorte que le chiffre 0 s'affiche sur les graduations 89 en regard du repère 97 du fourreau 92, on place le couteau 82 dans une position telle que sa pointe 83 soit placée en retrait par rapport à la surface de référence 13, d'une valeur telle que si, ensuite, on place par rotation du bouton de déclenchement 101 les nervures 102 en coïncidence avec les rainures 106 et les nervures 105 en coïncidence avec les nervures 103, il s'ensuive par action du ressort 98 une translation d'ensemble du bouton de déclenchement 101, du fourreau 92, du bouton de réglage 88 et du couteau 82, dans le sens 203, par rapport au porte-couteau 70 avec une amplitude telle que la pointe 83 vienne affleurer la surface de référence 13, comme on l'a illustré à la figure 1 ;
- et que, en plaçant à nouveau le bouton de déclenchement 101 en appui sur la zone extrême 95 du porte-couteau 70 par l'intermédiaire des méplats 104 et 107, et si l'on affiche alors au moyen du bouton de réglage 88, au moyen des graduations 89 et du repère 97, la saillie maximale envisagée pour la pointe 83 du couteau 82 par rapport à la surface de référence 93, la pointe 83 reste placée en retrait par rapport à la surface de référence 13 jusqu'à ce que l'on applique au bouton de déclenchement 101 une rotation telle que ses nervures et rainures correspondent respectivement avec les rainures et nervures de la zone extrême 95 du porte-couteau 70, le ressort 98 provoquant alors une translation brusque du bouton de déclenchement 101, du fourreau 92, du bouton de réglage 88 et du couteau 82, dans le sens 203, par rapport au porte-couteau 90, jusqu'à provoquer une saillie de la pointe 83 du couteau 82, par rapport à la surface de référence 13, d'une valeur correspondant à la valeur affichée au moyen du bouton de réglage 88.

Ainsi, le chirurgien peut, préalablement à la pose du kératotome 3 sur la face antérieure 1 de la cornée 2, pratiquer au moyen de la couronne 73 un réglage du décalage R₄ de l'intersection de la direction propre 71 avec la surface de référence 13, par rapport à l'axe 5, indépendamment pour l'un et l'autre des couteaux 82 et alors que la partie de tête 7 est démontée de la partie de corps 8 puis, après avoir remonté ces deux parties l'une sur l'autre, afficher au moyen du bouton de réglage 88 une profondeur d'incision pour chaque couteau 82, indépendamment, alors que ces couteaux 82 restent dans une position de repos, en retrait par rapport à la surface de référence 13. Après la mise en place sur la face antérieure de la cornée 2, en coïncidence de l'axe 5 avec l'axe visuel et après immobilisation du kératotome 3 par application d'une dépression dans la cavité 9, ou grâce à l'ancrage des dents 31 sur la cornée, le chirurgien peut en tournant le bouton de déclenchement 101 provoquer un passage brusque de chaque couteau 82 dans une position de saillie pré-réglée par rapport à la surface de référence 13, c'est-à-dire provoquer directement une pénétration de chaque couteau 82 sur une profondeur pré-réglée dans la cornée 2.

Ensuite, en agissant sur les moyens d'entraînement 61, le chirurgien provoque une rotation du corps 6 par rapport au support 4, ce qui se traduit par une rotation identique des deux couteaux 82 autour de l'axe visuel par rapport à la cornée 2, c'est-à-dire par la réalisation d'incisions arciformes de même longueur angulaire.

Pour permettre au chirurgien de contrôler, et de préférence de pré-régler, d'une part la position angulaire des incisions arciformes et d'autre part leur amplitude angulaire, le kératotome 3 selon l'invention présente des moyens de détermination de la position angulaire relative du support et du corps, autour de l'axe 5, lesquels moyens 108 vont être décrits à présent en référence aux figures 1, 2, 3 dans un mode de réalisation préféré.

Dans ce mode de réalisation, ces moyens 108 comportent notamment, dans la partie de tête 8 du support 4, une bague coaxiale 109 qui en est solidaire, avec possibilité de réglage de sa position angulaire.

A cet effet, la bague 109, réalisée en acier à ressort ou analogue présente une face périphérique extérieure 110 cylindrique d'un diamètre qui, lorsque cette bague est assemblée au support 4, est identique à celui de la face 44, que la bague 109 épouse par sa face 110 de la proximité immédiate de la face 58 de l'épaulement 49 du corps 6 jusqu'à la face 47. La bague 109 forme en outre une saillie au dessus de la face 47. A un niveau correspondant sensiblement à celui de la face 47, la face 110 est creusée d'une gorge annulaire continue 111 par laquelle elle s'emboîte sur une nervure annulaire, continue 112 que le support 4 présente, en saillie vers l'axe 5, par rapport à sa face 44, au raccordement de cette dernière avec la face 47.

La bague 109 est axialement limitée par une face annulaire plane 113, et par une arête circulaire 114 se raccordant à la face 113, par une face périphérique intérieure 116 pour l'essentiel tronconique de révolution autour de l'axe 5, sauf de façon localisée comme il apparaîtra plus loin.

La bague 109 présente une discontinuité sous forme d'une fente 117, ce qui la rend élastiquement compressible radialement, et elle est montée par rapport au support 4 en butée, par sa face 110, contre la face 44 du support 4 qui lui offre ainsi une portée de friction.

Toutefois, une sollicitation manuelle volontaire de la bague 109, en direction circonférentielle, dans le sens d'une fermeture de la fente 117, permet de réduire le diamètre de la face 110 de la bague 109, et par conséquent de provoquer à volonté la rotation de celle-ci autour de l'axe 5 par rapport au support 4 avant de la solidariser à nouveau avec celui-ci en relâchant ladite sollicitation manuelle.

En vue de permettre cette sollicitation, la bague 109 présente de part et d'autre de la fente 17, dans sa partie en saillie par rapport à la face 47 du support 4, des moyens de préhension manuelle sous la forme de deux encoches 118 laissant subsister entre elles et la fente 17 un ergot 119 offrant aux doigts du chirurgien un appui vers la fente 117.

Dans une zone périphérique intérieure 120 ne comprenant pas la fente 117 et présentant en une longueur angulaire α₃ de 180°, la face périphérique intérieure tronconique 116 ne se raccorde pas directement à la face 113 de la bague 109, et ce raccordement s'effectue par l'intermédiaire d'une face périphérique intérieure 121 cylindrique de révolution autour de l'axe 5, vers lequel cette face 121 est tournée. A ses deux extrémités, si l'on considère une direction circonférentielle centrée sur l'axe 5, la face 121 se raccorde par un épaulement de butée 122 à la face périphérique intérieure tronconique 116.

Complémentairement, le corps 6 porte réglable, en saillie parallèlement à l'axe 5 sur la face 58 de l'épaulement 49, dans une zone de cette face 58 qui reste dégagée par la bague 109, deux plots de butée 124 placés en regard de la zone périphérique intérieure 120 de la bague 109 et aptes à venir buter circonférentiellement contre l'un ou l'autre des épaulements 122, dans l'un ou l'autre sens 206, 207 de rotation du corps 6 par rapport au support 4. Ces plots de butée 124 sont symétriques par rapport au plan 204 dont ils sont respectivement décalés de moins de 90°, respectivement dans le sens 206 et dans le sens 207.

A cet effet, le corps 6 présente dans la face 58 une pluralité de trous borgnes 125 identiques, cylindriques de révolution autour d'axes respectifs 126 équirépartis angulairement autour de l'axe 5 et équidistants de celui-ci, et chacun de ces trous borgnes 125 peut recevoir l'un des plots de butée 124 ; dasn l'exemple préféré illustré, les trous borgnes 125 sont ainsi prévus au nombre de 13, répartis selon un pas angulaire α₄ de 10° sur un développement angulaire total de 120°, symétriquement par rapport au plan 204 ; par exemple, chaque trou 125 est taraudé et chaque plot 124 est pourvu d'une tige filetée 127 apte à se visser dans un trou 125.

En solidarisant les deux plots 124 avec le corps 6 dans des positions angulaires convenablement choisies en référence à l'axe 5, symétriques par rapport au plan 204, et dans la mesure où chaque plot 124 est propre à venir buter circonférentiellement contre l'un des épaulements 122 dans un sens 206, 207 de rotation du corps 6 par rapport au support 4, on peut fixer des limites de rotation du corps 6 autour de l'axe 5 par rapport au support 4 avec d'une part prédétermination de l'amplitude de rotation relative entre les orientations limites par réglage de la position angulaire des plots 124 sur le corps 6 et d'autre part, réglage du positionnement angulaire de ces orientations limites en référence au support 4 par réglage de la position angulaire des épaulements de butée 122 de la bague 109 par rapport à celui-ci.

A cet égard, les trous borgnes 125 jouent sur le corps 6 le rôle de graduation permettant de repérer visuellement la position angulaire de celui-ci par rapport à la bague 109, c'est-à-dire par rapport au support 4, en coopération avec les épaulements de butée 122 de la bague 109. De préférence, à chacun de ces trous borgnes 125 est en outre associée une graduation 123 qui, dans l'exemple illustré, est exprimée en valeur de décalage angulaire de l'axe 126 du trou borgne 125 considéré, par rapport au plan 205, autour de l'axe 5, exprimée en degrés d'angle. Ainsi, un trou borgne 125 dont l'axe 126 est situé dans le plan 204 porte à titre de graduation 123 le nombre 90 et les trous borgnes 125 qui se succèdent à partir de ce trou borgne 125, respectivement de part et d'autre du plan 204, portent les nombres 80, 70, 60, 50, 40, 30 respectivement.

Avant de provoquer la sortie brusque des couteaux 82 de leur position de repos à leur position de coupe et après avoir fixé le kératotome 3 en position convenable sur la face antérieure 1 de la cornée 2, il suffit au chirurgien d'amener, par action sur les moyens 61, le corps 6 dans une orientation dans laquelle il est en butée par l'un des plots 124 contre l'épaulement 122 correspondant puis, après avoir provoqué la sortie brusque des couteaux, de provoquer la rotation du corps 6 par rapport au support 4 jusqu'à ce que l'autre plot 124 vienne en butée contre l'autre épaulement 122, pour être assuré de réaliser des incisions d'une amplitude angulaire prédéterminée, et dans des positions également prédéterminées.

Pour faciliter les réglages préalables, le corps 6 porte également, de préférence, des moyens 128 de repérage d'un axe d'astigmatisme, par exemple sous une forme apte à être solidarisée avec le corps 6, de façon amovible, en même temps que celui des plots de butée 124 qui est décalé dans le sens 206 par rapport au plan 204 dans l'exemple illustré, par exemple par pincement entre ce plot et la face 58 de l'épaulement 49 du corps 1, de sorte que ces moyens de repérage se situent suivant un même plan incluant l'axe 5 et l'axe commun 126 de ce plot 124 et du trou 125 le recevant ; ce plan coïncide avec le plan repéré en IX-IX à la figure 2.

On a précisément illustré aux figures 2, 6, 7 des moyens 128 de repérage d'un axe d'astigmatisme ainsi conçus ; ces moyens se présentent sous la forme d'une aiguille 129 qui, comme le montre la figure 7, converge vers l'axe 5 dans un sens allant de la partie de tête 8 vers la partie de pied 7, tout en restant intégralement en retrait par rapport à la surface de référence 13.

Vers l'axe 5 et vers cette surface de référence 13, l'aiguille 129 présente un coude terminé par une extrémité pointue libre 130 qui aboutit à l'axe 5 au voisinage de la surface 13 de la référence de sorte que cette pointe constitue le repère à superposer à la marque de l'axe visuel du patient alors qu'à l'opposé, elle est emprisonnée de façon solidaire, par une autre extrémité 131, dans une pièce intermédiaire de montage 132 présentant une forme propre à épouser localement les faces 65, 67, 66 du corps 6 ainsi que la face 58 de l'épaulement 49 de celui-ci, et ceci par une zone fourchue 133 propre à s'intercaler entre le plot 124 respectif et la face 58, autour de la tige 127 de ce plot 124.

Dans ces conditions, le réglage de la longueur angulaire de chaque incision et sa position angulaire peuvent s'effectuer de la façon suivante, la profondeur et le diamètre d'incision étant supposés réglés et chaque couteau étant supposé occuper la position de repos :
- on détermine le développement angulaire de chacune des incisions à réaliser, en référence à l'axe visuel,
- on visse, dans les deux trous 125 dont les graduations 123 correspondent à la moitié du développement angulaire d'incision choisi, un plot 124 respectif en fixant les moyens 128 au moyen du plot 124 décalé dans le sens 206 par rapport au plan 104,
- on fait tourner la bague 109 par rapport au support 4 jusqu'à amener en contact le plot 124 muni des moyens 128 et l'épaulement de butée 122 associé, sans faire tourner le corps 6 par rapport au support 4,
- après avoir placé convenablement et immobilisé le kératotome sur la cornée, notamment en ayant placé en coincidence la pointe 13O et la marque de l'axe visuel du patient, en orientant les moyens 61 de manoeuvre manuelle d'une façon commode pour le chirurgien on fait éventuellement tourner conjointement la bague 109 et le corps 6 par rapport au support 4 en maintenant le contact précité du plot 124 muni des moyens 128 et de l'épaulement de butée 122 associé, pour amener l'aiguille 129 en coïncidence avec l'axe d'astigmatisme, repéré au préalable sur la face antérieure 1 de la cornée 2,
- on provoque le passage des couteaux à leur position de coupe et, sans faire tourner la bague 109 par rapport au support 4, on fait tourner le corps 6 par rapport à celui-ci dans le sens 207 jusqu'à ce que l'autre des plots 124 vienne au contact de l'autre des épaulements 122, ce qui réalise les incisions désirées,
- on rétracte les couteaux vers leur position de repos et on sépare le kératotome de la cornée.

Dans l'exemple illustré notamment à la figure 2, on réalise ainsi simultanément deux incisions arciformes d'un développement angulaire respectif de 120°C, équi-réparti de part et d'autre de l'axe d'astigmatisme, en plaçant les deux plots 124 dans les trous borgnes 125 repérés par les nombres 60 en tant que graduations 123 et, dans son mode de réalisation décrit, le kératotome selon l'invention, permet de réaliser ainsi une gamme d'incisions dont le développement angulaire respectif varie par pas de 20° entre 60°, auquel cas les plots 124 doivent être placés dans les trous borgnes 125 repérés par le nombre 30, et 180°, auquel cas un plot unique 124 doit être placé dans le trou borgne 125 repéré par le nombre 90, mais d'autre modes de variation des développements angulaires des incisions réalisables au moyen d'un kératotome selon l'invention de même que d'autres valeurs limites de ces développements angulaires pourraient être choisis sans que l'on sorte pour autant du cadre de la présente invention.

## Revendications

1. Kératotome destiné à la réalisation d'une incision dans une cornée et comportant à cet effet :
- un support extérieur tubulaire (4) formant enveloppe, présentant un axe (5) déterminé et comportant, pour son application sur une cornée (2), une partie de pied (7) annulaire de révolution autour de l'axe (5) du support (4) et délimitant une surface géométrique de référence (13) définie comme la forme que présente la cornée (2) lorsque le support (4) est appliqué sur celle-ci par sa partie de pied (7),
- un corps intérieur tubulaire (6) disposé coaxialement à l'intérieur du support (4), en retrait vers l'intérieur du support (4) par rapport à la surface de référence (13),
- des moyens (39, 50, 55, 56) de guidage du corps en rotation autour de l'axe (5) par rapport au support(4),
- des moyens (61) d'entraînement du corps (6) en rotation autour de l'axe (5) par rapport au support(4),
- un couteau (82) disposé à l'intérieur du corps (6), suivant une direction propre (71), et présentant dans un sens déterminé (203) de sa direction propre (71) une pointe coupante (83),
- des moyens (68, 70, 86) de liaison entre le couteau (82) et le corps (6), comportant eux-mêmes des moyens (87) pour déplacer de façon réglée le couteau (82) en translation suivant sa direction propre (71), par rapport au corps (6), entre une position de repos, dans laquelle il est placé en retrait vers l'intérieur du support (4) par rapport à la surface de référence (13) et dans laquelle la pointe (83) est tournée vers ladite surface de référence (13), et une position de coupe, dans laquelle la pointe (83) forme une saillie à l'extérieur du support (4) par rapport à la surface de référence (13), de façon décalée par rapport à l'axe (5) du support (4),
caractérisé en ce que, en vue de la réalisation d'incisions arciformes de position angulaire déterminée et de longueur angulaire déterminée, le support (4) et le corps (6) présentent des moyens (108) de détermination de leur position angulaire relative comprenant des butées (122, 124) définissant deux positions angulaires relatives limites du support (4) et du corps (6) et portées respectivement par le support (4) et par le corps (6).

2. Kératotome selon la revendication 1, caratérisé en ce que les moyens de détermination (108) comportent des moyens (122, 123) formant graduations de repérage de la position angulaire relative du support (4) et du corps (6) portés respectivement par le support (4) et par le corps (6).

3. Kératotome selon la revendication 1 ou la revendication 2, caractérisé en ce que le support (4) porte de façon réglable angulairement une bague coaxiale (109) portant des butées (122) de façon solidaire, décalées angulairement de 180° l'une par rapport à l'autre et en ce que le corps (6) porte des butées (124) réglabes angulairement de façon indépendante.

4. Kératotome selon la revendication 3, caractérisé en ce que le corps (6) porte de façon solidaire de l'une des butées (122) des moyens (128) de repérage d'un axe d'astigmatisme.

5. Kératotome selon l'une quelconque des revendications 3 et 4, caractérisé en ce que ladite bague (109) est ouverte par une fente (117) radiale, élastiquement compressible radialement et en butée radiale centrifuge contre une portée annulaire (44), coaxiale, du support (4) sous une précontrainte telle qu'elle soit solidarisée provisoirement avec le support (4) par friction mais que la fente (117) soit néanmoins ouverte et en ce qu'elle présente respectivement de part et d'autre de ladite fente radiale (117) des moyens de préhension (119) en vue d'une sollicitation manuelle en direction circonférentielle, dans le sens d'une fermeture de la fente (117).

6. Kératotome selon l'une quelconque des revendications 1 à 5, caractérisé en ce qu'il comporte en outre :
- un autre couteau (82) décalé angulairement de 180° par rapport au premier et disposé à l'intérieur du corps (6), suivant une direction propre (71) et présentant dans un sens déterminé (203) de sa direction propre (71) une pointe coupante (83),
- des moyens (68, 70) de liaison entre l'autre couteau (82) et le corps (6) comportant eux-mêmes des moyens (87) pour déplacer de façon réglée l'autre couteau (82) en translation suivant sa direction propre (71), par rapport au corps (6), entre une position de repos, dnas laquelle il est placé en retrait vers l'intérieur du support (4) par rapport à la surface de référence (13) et dans laquelle la pointe (83) est tournée vers ladite surface de référence (13) et une position de coupe, dans laquelle la pointe (83) forme une saillie à l'extérieur du support (4) par rapport à la surface de référence (13), de façon décalée par rapport à l'axe (5) du support (4).

7. Kératotome selon la revendication 6, caractérisé en ce que les moyens (87) pour déplacer de façon réglée chaque couteau (82) en translation suivant sa direction propre (71) comportent:
- des moyens (88, 89, 92, 97) pour afficher une valeur réglée de ladite saillie alors que le couteau (82) est en position de repos,
- des moyens (92, 98, 101) pour provoquer un passage volontaire brusque du couteau (82) de sa position de repos à sa position de coupe.

8. Kératotome selon la revendication 7, caractérisé en ce que les moyens (87) pour déplacer de façon réglée chaque couteau (82), en translation suivant sa direction propre (71) comportent :
- un bouton de réglage (88) placé en saillie hors du porte-couteau (70) à l'opposé de la pointe (83) monté en rotation autour de ladite direction propre (71) par rapport au porte-couteau (70), et solidaire en translation du couteau (82) suivant ladite direction propre (71),
- un fourreau (92) monté en translation par rapport au bouton de réglage (88) et par rapport au porte-couteau (70), suivant ladite direction propre (71), solidaire en rotation du porte-couteau (70) autour de ladite direction propre (71) et présentant un épaulement (99) transversal par rapport à ladite direction propre (71) et tourné dans un sens (203) allant de la position de repos vers la position de coupe,
- des moyens (90, 91) filetés de liaison entre le bouton (88) de réglage et le fourreau (92),
- des graduations micrométriques (89, 97) de repérage de la position angulaire relative du bouton de réglage (88) et du fourreau (92) autour de ladite direction propre (71), constituant lesdits moyens (88, 89, 92, 97) pour afficher une valeur réglée de ladite saillie,
- des moyens (98) de sollicitation élastique du fourreau (92) en translation par rapport au porte-couteau (70) suivant ladite direction (71) propre dans un sens (203) allant de la position de repos vers la position de coupe,
- un bouton de déclenchement (101) monté en rotation par rapport au bouton de réglage (88), au fourreau (92) et au porte-couteau (70), présentant un épaulement (100) transversal par rapport à ladite direction propre (71), tourné dans un sens (202) allant de la position de coupe vers la position de repos et en appui contre ledit épaulement transversal (99) du fourreau (92),
- des moyens (102, 103, 105, 106) de butée mutuelle du bouton de déclenchemnet (101) et du porte-couteau (70) suivant ladite direction propre (71), comportant sur le bouton de déclenchement (101) une alternance de nervures (102) et de rainures (102) radiales par rapport à ladite direction propre (71) et tournées dans un sens (203) allant de la position de repos vers la position de coupe et sur le porte-couteau (70) une alternance de rainures (106) et de nervures (105) radiales par rapport à ladite direction propre (71), tournées dans un sens (202) allant de la position de coupe vers la position de repos et respectivement complémentaires des nervures (102) et rainures (102) du bouton de déclenchement (101), lesdites nervures et rainures (102, 103, 105, 106) étant conformées de telle sorte qu'un appui mutuel du bouton de déclenchement (101) et du porte-couteau (70) par lesdites nervures (102, 105) comme par engagement-desdites nervures (102, 105) dans lesdites rainures (103, 106) soit stable.

9. Kératotome selon l'une quelconque des revendications 6 à 8,caractérisé en ce qu'il comporte des moyens (73, 78) de réglage de la valeur du décalage (R₄) de la pointe (83) de chaque couteau (82), par raport à l'axe (5) du support (4) en position de coupe, qui comportent :
- des moyens (68) d'articulation du porte-couteau (70) sur le corps (6), autour d'un axe déterminé (69) d'articulation décalé par rapport à l'axe (5) du support (4), en retrait vers l'intérieur du support (4) par rapport à la surface de référence (13), et perpendiculaire à la direction propre (71) du couteau (82) et à un plan (205) incluant l'axe (5) du support (4),
- des moyens (73, 78) pour immobiliser de façon réglée le porte-couteau (70) en rotation autour de l'axe d'articulation, par rapport au corps (6).

## Claims

1. A keratotome for making an incision in a cornea, and comprising, for this purpose:
an envelope-forming tubular outer support (4) having a determined axis (5) and including, for application against a cornea (2), an annular base portion (7) that is circularly symmetrical about the axis (5) of the support (4) and that defines a reference geometrical surface (13) determined by the shape presented by the cornea (2) when the support (4) has its base portion (7) pressed thereagainst;
a tubular inner body (6) disposed coaxially inside the support and set back towards the inside of the support (4) relative to the reference surface (13);
guide means (39, 50, 55, 56) for guiding the body in rotation about the axis (5) relative to the support (4);
drive means (61) for driving the body (6) in rotation about the axis (5) relative to the support (4);
a blade (82) disposed inside the body (6) and extending along a longitudinal direction (71), the blade having a sharp tip (83) in a determined direction (203) relative to its longitudinal direction (71); and
means (68, 70, 86) for providing a connection between the blade (82) and the body (6), said means including means (87) for displacing the blade (82) in controlled manner in translation along its longitudinal axis (71) relative to the body (6) between a rest position, in which it is set back towards the inside of the support (4) relative to the reference surface (13), and in which the tip (83) is directed towards said reference surface (13), and in a cutting position where the tip (83) projects outside from the support (4) relative to the reference surface (13), in a manner that is offset relative to the axis (5) of the support (4);
characterized in that in order to perform arcuate incisions of determined angular position and of determined angular length, the support (4) and the body (6) have determining means (108) for determining their relative angular position which include abutments (122, 124) defining two limiting relative angular positions between the support (4) and the body (6) and carried respectively by the support (4) and by the body (6).

2. A keratotome according to claim 1, characterized in that the determining means (108) include graduation forming means (122, 123) forming graduations for marking angular position relative to the support and the body (6) and carried respectively by the support (4) and by the body (6).

3. A keratotome according to claim 1 or claim 2, characterized in that the support (4) carries a coaxial ring (109) that is angularly adjustable in position and that carries abutments (122) that are integral therewith, the abutments being offset by 180° from each other, and in that the body (6) carries abutments (124) that are angularly adjustable independently.

4. A keratotome according to claim 3, characterized in that the body (6) is integral with one of the abutments (122) of the means (128) for marking the axis of astigmatism.

5. A keratotome according to claim 3 or 4, characterized in that said ring (109) is opened by a radial gap (117), which is elastically compressible radially and in centrifugal radial abutment against a coaxial annular bearing surface (44) of the support (4) under prestress such that it is temporarily secured to the support (4) by friction, but with the gap (117) nevertheless remaining open, and in that it has grasping means (119) on respective sides of said radial gap (117) for the purpose of enabling manual force to be applied circumferentially in such a direction as to close the gap (117).

6. A keratotome according to any one of claims 1 to 7, characterized in that it further includes:
another blade (82) angularly offset by 180° relative to the first blade and disposed inside the body (6) along its own axial direction (71) and having its own sharp tip (83) in a determined direction (203) along its longitudinal direction (71); and
link means (68, 70) between the other blade (82) and the body (6) themselves including means (87) for displacing the other blade (82) in adjustable manner in translation along its own longitudinal direction (71) relative to the body (6) between a rest position in which it is set back towards the inside of the support (4) relative to the reference surface (13), and in which its tip (83) points towards said reference surface (13), and a cutting position in which the tip (83) projects out from the support (4) relative to the reference surface (13) so as to be offset relative to the axis (5) of the support (4).

7. A keratotome according to claim 6, characterized in that the means (87) for displacing each of the blades (82) in adjustable manner in translation along its own longitudinal direction (71) comprise:
means (88, 89, 92, 97) for displaying the adjusted value of said projection while the blade (82) remains in its rest position; and
means (92, 98, 101) for causing the blade (82) to move voluntarily and suddenly from its rest position to its cutting position.

8. A keratotome according to claim 7, characterized in that the means (87) for displacing each blade (82) in adjustable manner in translation along its own longitudinal direction (71) comprise:
an adjustment knob (88) placed to project beyond the blade carrier (70) away from the tip (83) and mounted to rotate about said longitudinal direction (71) relative to the blade carrier (70), and constrained to move in translation with the blade (82) along said longitudinal direction (71);
a sleeve (92) mounted to move in translation relative to the adjustment knob (88) and relative to the blade carrier (70), along said longitudinal direction (71), and constrained to rotate with the blade carrier (70) about said longitudinal direction (71), and having a transverse shoulder (99) extending transversely relative to said longitudinal direction (71) and facing in a direction (203) going from the rest position towards the cutting position;
threaded connection means (90, 91) between the adjustment knob (88) and the sleeve (92);
micrometer graduations (89, 97) for identifying the relative angular position of the adjustment knob (88) and of the sleeve (92) about said longitudinal direction (71), and constituting said means (88, 89, 92, 97) for displaying the adjusted value of said projection;
means (98) for resiliently urging the sleeve (92) in translation relative to the blade carrier (70) along said longitudinal direction (71) in a direction (203) going from the rest position towards the cutting position;
a trip knob (101) mounted to rotate relative to the adjustment knob (88), to the sleeve (92), and to the blade carrier (70), and having a shoulder (100) extending transversely relative to said longitudinal direction (71) and facing in a direction (202) going from the cutting position towards the rest position, and bearing against said transverse shoulder (99) of the sleeve (92); and
mutual abutment means (102, 103, 105, 106) between the trip knob (101) and the blade carrier (70) in said longitudinal direction (71), comprising on the trip knob (101) alternating radial ribs (102) and grooves (103) about said longitudinal direction (71) and facing in a direction (203) going from the rest position towards the cutting position, and on the blade carrier (70) alternating radial grooves (106) and ribs (105) about said longitudinal direction (71), and facing in a direction (202) going from the cutting position towards the rest position, and respectively complementary to the ribs (102) and the grooves (103) of the trip knob (101), said ribs and grooves (102, 103, 105, 106) being shaped so that mutual thrust between the trip knob (101) and the blade carrier (70) by means of said ribs (102, 105) as by engagement of said ribs (102, 105) in said grooves (103, 106) is stable.

9. A keratotome according to any one of claims 6 to 8, characterized in that it includes means (73, 78) for adjusting the offset value (R₄) of the tip (83) of each blade (82) relative to the axis (5) of the support (4) in the cutting position, which means comprise:
hinge means (68) for hinging the blade carrier (70) to the body (6) about a determined hinge axis (69) offset relative to the axis (5) of the support (4) and set back towards the inside of the support (4) relative to the reference surface (13), and perpendicular to the longitudinal direction (71) of the blade (82) and to a plane (205) including the axis (5) of the support (4); and
means (73, 78) for locking the blade carrier (70) relative to the body (6) in adjustable manner in rotation about the hinge axis.

## Patentansprüche

1. Keratotom, das zur Durchführung eines Hornhautschnittes bestimmt ist und zu diesem Zweck umfaßt:
ein ein Gehäuse bildendes äußeres rohrförmiges Trägerteil (4), das eine festgelegte Achse (5) und zum Aufsetzen auf eine Hornhaut (2) um die Achse (5) einen kreisringförmigen Fußbereich (7) hat und eine geometrische Bezugsfläche (13) begrenzt, die als die Form definiert wird, welche die Hornhaut (2) zeigt, wenn das Trägerteil (4) mit seinem Fußbereich (7) auf die Hornhaut aufgesetzt wird;
einen inneren Rohrkörper (6), der in dem Trägerteil (4) koaxial und relativ zur Bezugsfläche (13) nach innen in das Trägerteil (4) zurückgezogen angeordnet ist;
Mittel (39,50,55,56) zur Drehführung des Körpers um die Achse (5) relativ zu dem Trägerteil (4);
Mittel (61) zum Drehantrieb des Körpers (6) um die Achse (5) relativ zu dem Trägerteil (4);
ein Messer (82), das in einer geeigneten Richtung (71) in dem Körper (6) angeordnet ist und nach einer bestimmten Seite (203) dieser Richtung (71) eine Schneidspitze (83) besitzt;
Mittel (68,70,86) für eine Verbindung zwischen dem Messer (82) und dem Körper (6), die ihrerseits Mittel (87) umfassen zur gesteuerten translatorischen Bewegung des Messers (82) in seiner geeigneten Richtung (71) relativ zu dem Körper (6) zwischen einer Ruheposition, in der es relativ zur Bezugsfläche (13) nach innen in das Trägerteil zugsfläche (13) zugewandt ist, und einer Schneidposition, in der die Spitze (83) relativ zur Bezugsfläche (13) aus dem Trägerteil (4) vorspringt, derart, daß sie relativ zur Achse (5) des Trägerteils (4) versetzt ist;
dadurch gekennzeichnet, daß das Trägerteil (4) und der Körper (6) zur Durchführung kreisbogenförmiger Einschnitte mit einer bestimmten Winkelposition und Winkellänge Mittel (108) zur Bestimmung ihrer relativen Winkelposition besitzen, die Anschläge (122,124) umfassen, die zwei relative Winkelendpositionen des Trägerteils (4) und des Körpers (6) definieren und jeweils von dem Trägerteil (4) und von dem Körper (6) getragen sind.

2. Keratotom nach Anspruch 1, dadurch gekennzeichnet, daß die Bestimmungsmittel (108) Mittel (122,123) umfassen, die jeweils von dem Trägerteil (4) und von dem Körper (6) getragene Gradeinteilungen zum Markieren der relativen Winkelposition des Trägerteils (4) und des Körpers (6) bilden.

3. Keratotom nach Anspruch 1 oder 2, dadurch gekennzeichnet, daß das Trägerteil (4) einen im Winkel verstellbaren koaxialen Ring (109) trägt, der mit ihm verbundene Anschläge (122) hat, die relativ zueinander um einem Winkel von 180° versetzt sind, und daß der Körper (6) Anschläge (124) trägt, die in unabhängiger Weise im Winkel verstellbar sind.

4. Keratotom nach Anspruch 3, dadurch gekennzeichnet, daß der Körper (6) mit dem einen der Anschläge (122) verbundene Mittel (128) zum Markieren einer Astigmatismusachse trägt.

5. Keratotom nach einem der Ansprüche 3 und 4, dadurch gekennzeichnet, daß der Ring (109) durch einen radialen Schlitz (117) unterbrochen ist, in radialer Richtung elastisch zusammengedrückt werden kann und nach radial außen gegen eine koaxiale ringförmige Lagerfläche (44) des Trägers (4) unter einer Vorspannung anliegt derart, daß er durch Reibung provisorisch mit dem Trägerteil (4) verbunden ist, der Schlitz (117) aber dennoch offen ist und daß er beidseits des radialen Schlitzes (117) jeweils Greifmittel (119) für eine in Umfangsrichtung wirkende manuelle Beaufschlagung in der Schließrichtung des Schlitzes (117) besitzt.

6. Keratotom nach einem der Ansprüche 1 bis 5, ferner gekennzeichnet durch:
ein weiteres Messer (82), das relativ zu dem ersten Messer um einen Winkel von 180° versetzt und in einer geeigneten Richtung (71) in dem Körper (6) angeordnet ist und nach einer bestimmten Seite (203) dieser Richtung (71) eine Schneidspitze (83) hat;
Mittel (68,70) für eine Verbindung zwischen dem anderen Messer (82) und dem Körper (6), die ihrerseits Mittel (87) umfassen für eine gesteuerte translatorische Bewegung des anderen Messers (82) in seiner geeigneten Richtung (71) relativ zu dem Körper (6) zwischen einer Ruheposition, in der es relativ zur Bezugsfläche (13) nach innen in das Trägerteil (4) zurückgezogen ist und in der die Spitze (83) der Bezugsfläche (13) zugewandt ist, und einer Schneidposition, in der die Spitze (83) relativ zur Bezugsfläche (13) aus dem Trägerteil (4) vorspringt, derart, daß sie relativ zur Achse (5) des Trägerteils (4) versetzt ist.

7. Keratotom nach Anspruch 6, dadurch gekennzeichnet, daß die Mittel (87) zur gesteuerten translatorischen Bewegung jedes Messers (82) in seiner geeigneten Richtung (71) umfassen:
Mittel (88,89,92,97) zur Anzeige des eingestellten Wertes des genannten Vorsprungs, während sich das Messer (82) in der Ruheposition befindet;
Mittel (92,98,101), die eine beabsichtigte schlagartige Bewegung des Messers (82) aus seiner Ruheposition in seine Schneidposition bewirken.

8. Keratotom nach Anspruch 7, dadurch gekennzeichnet, daß die Mittel (87) für die gesteuerte translatorische Bewegung jedes Messers (82) in seiner geeigneten Richtung (71) umfassen:
einen Einstellknopf (88), der nach der der Spitze (83) entgegengesetzten Seite über den Messerhalter (70) hervorstehend angeordnet, relativ zu dem Messerhalter (70) um die geeignete Richtung (71) drehbar gelagert und in dieser Richtung (71) gemeinsam mit dem Messer (82) translatorisch bewegbar ist;
eine Hülse (92), die relativ zu dem Einstellknopf (88) und zu dem Messerhalter (70) in der genannten geeigneten Richtung (71) translatorisch bewegbar gelagert und mit dem Messerhalter (70) um die geeignete Richtung (71) drehbar drehfest verbunden ist und eine Schulter (99) hat, die quer zu der geeigneten Richtung (71) angeordnet ist und in eine aus der Ruheposition in die Schneidposition führende Richtung (203) weist;
mit einem Gewinde versehene Mittel (90,91) für eine Verbindung zwischen dem Einstellknopf (88) und der Hülse (92);
mikrometrische Gradeinteilungen (89,97) zum Markieren der relativen Winkelposition des Einstellknopfes (88) und der Hülse (92) um die genannte geeignete Richtung (71), wobei diese Gradeinteilungen die genannten Mittel (88,89,92,97) diese Gradeinteilungen die genannten Mittel (88,89,92,97) zum Anzeigen eines eingestellten Wertes des genannten Vorsprungs bilden;
Mittel (98) zum elastischen translatorischen Vorspannen der Hülse (92) relativ zu dem Messerhalter (70) entlang der geeigneten Richtung (71) in einem aus der Ruheposition in die Schneidposition führenden Richtungssinn (203);
einen relativ zu dem Einstellknopf (88) an der Hülse (92) und an dem Messerhalter (70) drehbar gelagerten Auslöseknopf (101), der eine quer zu der genannten geeigneten Richtung (71) gerichtete Schulter (100) hat, die in eine aus der Schneidposition in die Ruheposition führende Richtung weist und an der genannten Querschulter (99) der Hülse (92) anliegt;
Mittel (102,103,105,106) für die gegenseitige Anlage des Auslöseknopfes (101) und des Messerhalters (70) in der geeigneten Richtung (71), wobei diese Mittel an dem Auslöseknopf (101) eine alternierende Folge von Rippen (102) und Nuten (103) umfassen, die in bezug auf die genannte Richtung (71) radial angeordnet sind und in eine aus der Ruheposition in die Schneidposition führende Richtung (203) weisen, und wobei diese Mittel an dem Messerhalter (70) eine alternierende Folge von Nuten (106) und Rippen (105) umfassen, die in bezug auf die genannte geeignete Richtung (71) radial angeordnet sind und in eine aus der Schneidposition in die Ruheposition führende Richtung (202) weisen und die jeweils komplementär zu den Rippen (102) und Nuten (103) des Auslöseknopfes (101) ausgebildet sind, wobei die Rippen und Nuten (102,103,105,106) einander so entsprechen, daß eine gegenseitige Anlage des Auslöseknopfes (101) und des Messerhalters (70) über die genannten Rippen (102,105) wie über den Eingriff dieser Rippen (102,105) in die Nuten (103, 106) stabil ist.

9. Keratotom nach einem der Ansprüche 6 bis 8, dadurch gekennzeichnet, daß es Mittel (73,78) zur Einstellung des Versatzes (R₄) der Spitze (83) jedes Messers (82) relativ zur Achse (5) des Trägerteils (4) in der Schneidposition hat, wobei diese Mittel umfassen:
Mittel (68) zur Anlenkung des Messerhalters (70) an dem Körper (6) um eine relativ zur Achse (5) des Trägerteils (4) versetzte, in einer relativ zu der Bezugsfläche (13) nach innen in das Trägerteil (4) zurückgezogenen Position festgelegte Gelenkachse (69), die senkrecht zur Richtung (71) des Messers (82) und zu einer Ebene (205) ist, die die Achse (5) des Trägerteils (4) enthält;
Mittel (73,78) zum einstellbaren Festlegen des um die Gelenkachse drehbaren Messerhalters (70) relativ zu dem Körper (6).
